# EUROPEAN PATENT SPECIFICATION

(11) **EP 0 944 640 B1**
(45) Date of publication and mention of the grant of the patent: **22.05.2002**
(21) Application number: 97953718.0
(22) Date of filing: 20.11.1997
(51) Int. Cl.: C07H 15/02, C07H 15/08, B01F 17/00, C11D 17/00

(54) **USE OF A CRYSTALLISATION-INHIBITING ADDITIVE FOR SURFACE-ACTIVE NONIONIC GLYCOSIDES**
VERWENDUNG VON ADDITIVEN ALS KRISTALLISATIONSINHIBITOR FÜR NICHTIONISCHE GLYKOSIDE
UTILISATION D'UN ADDITIF INHIBANT LA CRISTALLISATION POUR LES GLYCOSIDES NON IONIQUES TENSIOACTIFS

(30) Priority: 11.12.1996 SE 9604546
(43) Date of publication of application: 29.09.1999
(73) Proprietor: AKZO NOBEL N.V., 6800 SB Arnhem (NL)
(72) Inventor: JOHANSSON, Ingegärd, S-416 54 Göteborg (SE); KARLSSON, Bo, S-471 32 Skärhamn (SE)
(74) Representative: Andersson, Rolf
(86) International application number: EP9706669
(87) International publication number: WO9825941

(56) References cited:
- WO-A-95/04592
- DE-A- 3 918 135
- US-A- 5 001 114
- DATABASE WPI Section Ch, Week 9420 Derwent Publications Ltd., London, GB; Class D21, AN 94-164276 XP002059877 & JP 06 108 091 A (KANEBO LTD) , 19 April 1994

## Description

The present invention relates to a concentrated surface-active glycoside composition, which contains an anionic derivative of an alkyl glycoside or a nonionic alkylene oxide adduct of an alkyl glycoside as a crystallisation-inhibiting additive.

It is well-known that concentrated solutions of alkyl polyglycosides have an undesired tendency to precipitate crystals when stored for a long time. The alkyl glycosides, such as α-alkyl glycosides, have a steric appearance, which makes them easily form crystals with crystal water. Since the formation of crystals is disadvantageous in many respects, it is important to reduce the crystallisation as much as possible.

From WO 94/03569 it is known that the crystallisation of alkyl or alkylene oligoglycosides can be reduced by adding alkyl glycosides on the basis of short-chain alcohols, oligoglycosides acetalised with guerbet alcohols or polyols, short-chain primary alcohols, fatty alcohol polyglycol ethers, polyethylene glycol, glucose or Fe-(III)-ions. WO 95/04592 suggests the addition of a surface-active amphoteric compound to an alkyl polyglycoside having an alkyl chain of at least 10 carbon atoms for preventing crystallisation, whereas DE 40 33 928 discloses the use of a partially esterified glyceride for the same purpose.

Even if the crystallisation-inhibiting additives, which are described in the above-mentioned patent publications, reduce the crystallisation, the effect is in many cases insufficient, especially after a long time of storing. Besides, the additives may change the properties of the glycosides in an undesirable manner. For example, the frothing of the alkyl glycosides may be affected.

According to the present invention, it is therefore desired that the crystallisation-reducing additives, besides having a high crystallisation-inhibiting capacity, should also be active in relative small amounts and, in these amounts, have little effect on the properties of the glycosides.

According to the present invention, it has now surprisingly been found that
a) an anionic derivative of a glycoside of the formula

   R(A)ₛ(G)ₙ (I)

   wherein R is an aliphatic hydrocarbon group having 6-20, preferably 8-16 carbon atoms, G is a saccharide residue, A is an oxyalkylene having 2-4 carbon atoms or 3-oxy-2--hydroxypropylene, n is a number from 1 to 10, preferably a number from 1 to 4, and s is a number from 0 to 5; or
b) a nonionic alkylene oxide adduct of a glycoside of formula I, wherein the alkylene oxide contains 2-4 carbon atoms and has an excellent crystallisation-inhibiting effect on an aqueous concentrate of a glycoside of formula I. Owing to the fact that the crystallisation-inhibiting additive is based on a glycoside of the same fundamental structure, it has been found that the changes of the properties of the glycoside, that are caused by the additive, can be reduced to a considerable extent.

The group G is suitably a monosaccharide residue, such as pentose or hexose. Specific examples are glucose, mannose, galactose, talose, allose, altrose, idose, arabinose, xylose, ribose and lyxose. For commercial reasons, glucose is preferred. The group R can be a straight or branched, saturated or unsaturated aliphatic group. Examples of suitable aliphatic groups R are obtained by reaction with coconut alcohols, n-hexyl alcohol, n-octanol, n-decyl alcohol, n-dodecyl alcohol, n-tetradecyl alcohol, n-hexadecyl alcohol, n-octadecyl alcohol, undecyl alcohol, tridecyl alcohol, oxo and guerbet alcohols, such as 2-ethylhexanol, 2-propylheptanol, 2-butyloctanol and methyl-substituted alcohols containing 2-5 groups of the formula -CH(CH₃)- in the carbon chain. According to a preferred embodiment, the group R in the additive and the hydrophobic group in the surface-active glycoside are equal.

The anionic derivatives are preferably carboxylates, but also sulphonates, phosphates, phosphonates and sulphates can be used. The number of anionic groups is usually 1 or 2.

The nonionic, crystallisation-inhibiting alkylene oxide adduct is suitably an adduct, which is obtained by adding 1-10, preferably 2-6 alkyleneoxy units per mole of alkyl glycoside. By selecting different alkylene oxides or combinations thereof, the frothing can easily be adapted to a glycoside of formula I. For instance, adducts with propylene or butylene oxide, or a combination of ethylene oxide and of higher alkylene oxide in the end position result in lower frothing than an adduct with only ethylene oxide. The additives can be added in amounts of about 0.1-20% by weight, based on the weight of the surface-active alkyl glycoside, which usually is 10-80% of the concentrate.

Preferred anionic derivatives are those exhibiting the general formula 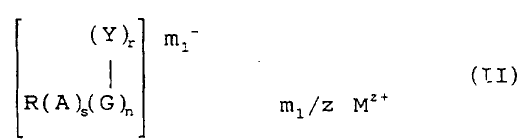 wherein R, A, G, s and n are as above, r is a number 0 or 1, the sum of all r being at least 1, preferably 1-2, Y is a group (OR₁)ₚ(X)ₘ, wherein R₁ is an aliphatic hydrocarbon group having 1-8, preferably 1-4 carbon atoms, m is a number 1-2, X is a carboxyl-containing group, p is 0 or 1, and m is 1, m₁ is the total charge of the anion and M is a preferably monovalent cation, such as a sodium ion, and z is the charge of the cation. Compounds of formula II can be prepared in the manner stated in WO 88/01640 and DE 40 15 655.

Preferred nonionic ethylene oxide adducts are those having the formula 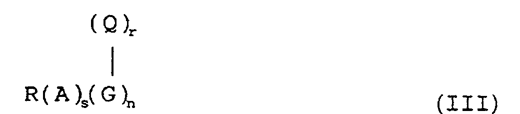 wherein R, A, G, s, n and r are as above and Q is the group (B)ᵥH, wherein B is an alkyleneoxy group and v is a number from 1 to 12, preferably 2 to 6. Compounds of formula III can suitably be prepared in a prior-art manner by alkoxilating a compound of formula I.

The present invention also relates to a concentrate containing
a) 10-80, preferably 45-70% by weight of an alkyl glycoside of formula I,
b) 0.1-20, preferably 0.5-15% by weight, based on the amount of alkyl glycoside, of the crystallisation-inhibiting additive according to the invention, and
c) 4-90% by weight of water.

If the crystallisation-inhibiting additive is an anionic derivative, the concentrate suitably contains 0.1-8, preferably 0.5-5% by weight, based on the alkyl glycoside, whereas when the crystallisation-inhibiting additive is a nonionic alkylene oxide adduct, the content suitably is 2-20, preferably 3-15% by weight, based on the alkyl glycoside. The concentrate may also contain solubilising agents, for instance propylene glycol, for improving the solubility, especially at low temperatures.

The present invention is further illustrated by the following Example.

### Example

Aqueous concentrates designated 1-6 and formulated according to the invention and designated A-D as comparative compositions were prepared by mixing in water the components stated in Table 1.

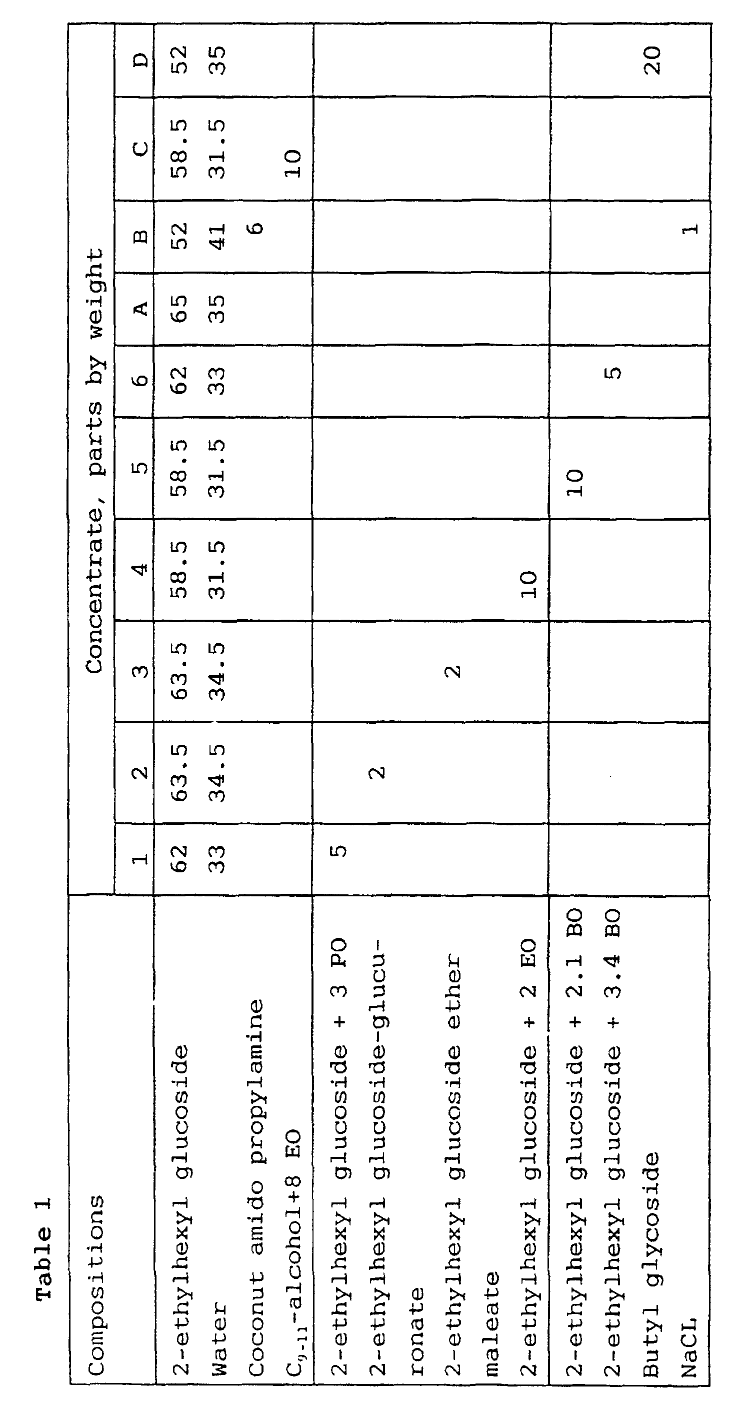

The formulations according to 1-6 and A-D where then allowed to stand from 2 to 6 months at 20°C and 6°C and any crystallisations were noted. The formulations were also tested in respect of frothing (Ross-Miles, after 0 min and 5 min at a concentration of 0.05% by weight at 20°C) and surface tension at a concentration of 0.1% by weight at 20°C. The following results were obtained.

**Table 2**

| Formulation | Appearance, crystallisation | | Frothing | | Surface tension mN/m |
|---|---|---|---|---|---|
| | 20°C | 6°C | 0 min | 5 min | |
| A | 3 days | 24 h | 28 | 0 | 36.0 |
| B | > 2 months | > 2 months | 95 | 75 | 30.0 |
| C | 3 days | 24 h | 80 | 57 | 31.4 |
| D | > 3 months | > 3 months | 45 | 0 | 38.0 |
| 1 | > 6 months | > 2 months | 20 | 0 | 35.5 |
| 2 | > 6 months | > 2 months | 27 | 0 | 35.5 |
| 3 | > 3 months | > 2 months | 40 | 0 | 37.3 |
| 4 | > 5 months | > 5 months | 9 | 0 | 36.0 |
| 5 | > 5 months | > 5 months | 12 | 0 | 36.0 |
| 6 | > 5 months | > 5 months | 8 | 0 | 34.9 |

As appears from the results, the formulations B-D had either a considerably increased degree of frothing (B and C) or a lower reduction of the surface tension (D). The formulations 1-6 according to the invention have approximately the same degree of frothing and surface tension as the formulation A, which has no addition of a crystallisation-inhibiting compound.

## Claims

1. Use of an anionic derivative of a glycoside of the formula
R(A)ₛ(G)ₙ (I)
wherein R is an aliphatic hydrocarbon group having 6-20, preferably 8-16 carbon atoms, G is a saccharide residue, A is an oxyalkylene having 2-4 carbon atoms or 3-oxy-2--hydroxypropylene, n is a number from 1 to 10, preferably a number from 1 to 4, and s is a number from 0 to 5; or a nonionic alkylene oxide adduct of a glycoside of formula I, wherein the alkylene oxide contains 2-4 carbon atoms, for inhibiting or preventing crystallisation of an aqueous concentrate of a glycoside of formula I.

2. Use as claimed in claim 1, wherein the anionic derivative is a carboxylate, sulphonate, phosphate, phosphonate or sulphate, and the nonionic alkylene oxide adduct is obtained by adding 1-10, preferably 2-6 alkyleneoxy units per mole cf alkyl glycoside.

3. Use as claimed in any one of claims 1-2, wherein the anionic derivative and/or the nonionic alkylene oxide adduct is used in an amount of 0.1-20% by weight, based on the weight of the alkyl glycoside.

4. Use as claimed in any one of claims 1-3, wherein the anionic derivative is of the general formula 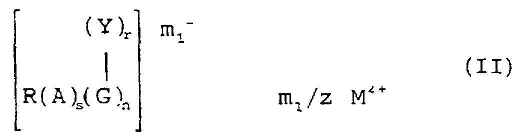 wherein R, A, G, s and n are as above, r is a number 0 or 1, the sum of all r being at least 1, preferably 1-2, Y is a group (OR₁)ₚ(X)ₘ, wherein R₁ is an aliphatic hydrocarbon group having 1-8, preferably 1-4 carbon atoms, m is a number 1-2, X is a carboxyl-containing group, p is 0 or 1, and m is 1, m₁ is the total charge of the anion and M is a preferably monovalent cation, and z is the charge of the cation.

5. Use as claimed in any one of claims 1-4, **characterised in that** the ethylene oxide adduct is of formula 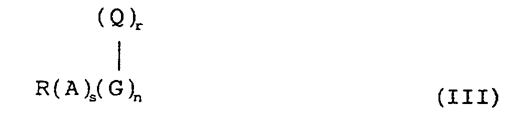 wherein R, A, G, s, n and r are as above and Q is the group (B)ᵥH, wherein B is an alkyleneoxy group and v is a number from 1 to 12, preferably 2 to 6.

6. Use as claimed in any one of claims 1-5, wherein R in the glycoside and the anionic derivative or the nonionic alkylene oxide adduct are equal.

7. A concentrate of an alkyl glycoside, **characterised in that** it contains
a) 10-80, preferably 45-70% by weight of an alkyl glycoside of formula I according to claim 1,
b) 0.1-20, preferably 0.5-15% by weight, based on the alkyl glycoside, of a crystallisation-inhibiting additive as claimed in claim 1, 2, 4, 5 or 6, and
c) 4-90% by weight of water.

8. A concentrate as claimed in claim 7, **characterised in that** the crystallisation-inhibiting additive consists of the anionic derivative, which has a content of 0.1-8% by weight, based on the alkyl glycoside.

9. A concentrate as claimed in claim 7, **characterised in that** the crystallisation-inhibiting derivative consists of the nonionic alkylene oxide adduct, which has a content of 2-20% by weight, based on the alkyl glycoside.

10. A method of preventing or inhibiting the forming of crystallisation in an aqueous concentrate of an alkyl glycoside, **characterised in that** an anionic derivative of a glycoside of formula
R(A)ₛ(G)ₙ (I)
wherein R is an aliphatic hydrocarbon group having 6-20, preferably 8-16 carbon atoms, G is a saccharide residue, A is an oxyalkylene having 2-4 carbon atoms or 3-oxy-2--hydroxypropylene, n is a number from 1 to 10, preferably a number from 1 to 4, and s is a number from 0 to 5; or a nonionic alkylene oxide adduct, wherein the alkylene oxide contains 2-4 carbon atoms, of a glycoside of formula I, is added to the concentrate in an amount of 0.1-20% by weight, based on the weight of the alkyl glycoside.

## Patentansprüche

1. Verwendung eines anionischen Derivats eines Glycosids der Formel
R(A)ₛ(G)ₙ (I)
worin R eine aliphatische Kohlenwasserstoffgruppe mit 6 bis 20, vorzugsweise 8 bis 16 Kohlenstoffatomen ist, G ein Saccharidrest ist, A ein Oxyalkylen mit 2 bis 4 Kohlenstoffatomen oder 3-Oxy-2-hydroxypropylen ist, n eine Zahl von 1 bis 10, vorzugsweise eine Zahl von 1 bis 4 ist und s eine Zahl von 0 bis 5 ist, oder eines nicht-ionischen Alkylenoxidaddukts eines Glycosids der Formel I, worin das Alkytenoxid 2 bis 4 Kohlenstoffatome enthält, zur Hemmung oder Vermeidung der Kristallisation in einem wäßrigen Konzentrat eines Glycosids der Formel I.

2. Verwendung wie in Anspruch 1 beansprucht, worin das anionische Derivat ein Carboxylat, Sulfonat, Phosphat, Phosphonat oder Sulfat ist und das nicht-ionische Alkylenoxidaddukt durch Zugabe von 1 bis 10, vorzugsweise 2 bis 6 Alkylenoxy-Einheiten pro Mol Alkylglycosid erhalten wird.

3. Verwendung wie in irgendeinem der Ansprüche 1 bis 2 beansprucht, worin das anionische Derivat und/oder das nicht-ionische Alkylenoxidaddukt in einer Menge von 0,1 bis 20 Gew.-%, bezogen auf das Gewicht des Alkylglycosids, verwendet wird.

4. Verwendung wie in irgendeinem der Ansprüche 1 bis 3 beansprucht, worin das anionische Derivat die allgemeine Formel 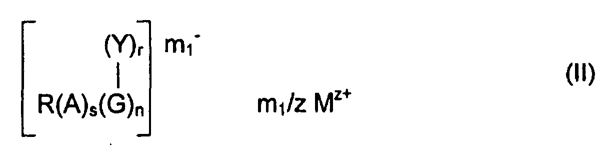 hat, worin R, A, G, s und n wie vorstehend sind, r die Zahl 0 oder 1 ist, wobei die Summe aller r mindestens 1, vorzugsweise 1 bis 2, ist, Y eine Gruppe (OR₁)ₚ(X)ₘ ist, worin R₁ eine aliphatische Kohlenwasserstoffgruppe mit 1 bis 8, vorzugsweise 1 bis 4 Kohlenstoffatomen ist, m eine Zahl von 1 bis,2 ist, X eine Carboxyl enthaltende Gruppe ist, p 0 oder 1 ist und m 1 ist, m₁ die Gesamtladung des Anions ist und M ein vorzugsweise einwertiges Kation ist und z die Ladung des Kations darstellt.

5. Verwendung wie in irgendeinem der Ansprüche 1 bis 4 beansprucht, **dadurch gekennzeichnet, daß** das Ethylenoxidaddukt die Formel 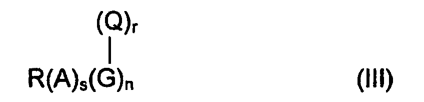 aufweist, worin R, A, G, s, n und r wie oben sind und Q die Gruppe (B)ᵥH ist, worin B eine Alkylenoxygruppe ist und v eine Zahl von 1 bis 12, vorzugsweise 2 bis 6, ist.

6. Verwendung wie in irgendeinem der Ansprüche 1 bis 5 beansprucht, worin R in dem Glycosid und dem anionischen Derivat oder dem nicht-ionischen Alkylenoxidaddukt gleich ist.

7. Konzentrat eines Alkylglycosids, **dadurch gekennzeichnet, daß** es enthält
a) 10 bis 80 Gew.-%, bevorzugt 45 bis 70 Gew.-%, eines Alkylglycosids der Formel I nach Anspruch 1,
b) 0,1 bis 20 Gew.-%, bevorzugt 0,5 bis 15 Gew.-%, bezogen auf das Alkylglycosid, eines kristallisationshemmenden Additivs wie in Anspruch 1, 2, 4, 5 oder 6 beansprucht, und
c) 4 bis 90 Gew.-% Wasser.

8. Konzentrat wie in Anspruch 7 beansprucht, **dadurch gekennzeichnet, daß** das kristallisationshemmende Additiv aus dem anionischen Derivat besteht, das einen Gehalt von 0,1 bis 8 Gew.-%, bezogen auf das Alkylglycosid, aufweist.

9. Konzentrat wie in Anspruch 7 beansprucht, **dadurch gekennzeichnet, daß** das kristallisationshemmende Derivat aus dem nicht-ionischen Alkylenoxidaddukt besteht, das einen Gehalt von 2 bis 20 Gew.-%, bezogen auf das Alkylgylcosid, aufweist.

10. Verfahren zur Vermeidung oder Hemmung der Kristallisationsbildung in einem wäßrigen Konzentrat eines Alkylglycosids, **dadurch gekennzeichnet, daß** ein anionisches Derivat eines Glycosids der Formel
R(A)ₛ(G)ₙ (I)
worin R eine aliphatische Kohlenwasserstoffgruppe mit 6 bis 20, vorzugsweise 8 bis 16 Kohlenstoffatomen ist, G ein Saccharidrest ist, A ein Oxyalkylen mit 2 bis 4 Kohlenstoffatomen oder 3-Oxy-2-hydroxypropylen ist, n eine Zahl von 1 bis 10, vorzugsweise eine Zahl von 1 bis 4 ist und s eine Zahl von 0 bis 5 ist, oder ein nicht-ionisches Alkylenoxidaddukt, worin das Alkylenoxid 2 bis 4 Kohlenstoffatome enthält, eines Glycosids der Formel I zum Konzentrat in einer Menge von 0,1 bis 20 Gew.-%, bezogen auf das Gewicht des Alkylglycosids, zugegeben wird.

## Revendications

1. Utilisation d'un dérivé anionique d'un glycoside dé formule
R(A)ₛ(G)ₙ (I)
dans laquelle R est un groupe hydrocarboné aliphatique comportant de 6 à 20, de préférence de 8 à 16 atomes de carbone, G est un résidu saccharide, A est un oxyalkylène comportant de 2 à 4 atomes de carbone ou un 3-oxy-2-hydroxypropylène, n est un nombre allant de 1 à 10, de préférence un nombre allant de 1 à 4, et s est un nombre allant de 0 à 5 ; ou un produit d'addition d'oxyde d'alkylène non ionique d'un glycoside de formule I, dans lequel l'oxyde d'alkylène contient 2 à 4 atomes de carbone, pour inhiber ou prévenir la cristallisation d'un concentré aqueux d'un glycoside de formule I.

2. Utilisation selon la revendication 1, dans laquelle le dérivé anionique est un carboxylate, sulfonate, phosphate, phosphonate ou sulfate, et le produit d'addition d'oxyde d'alkylène non ionique est obtenu en ajoutant de 1 à 10, de préférence de 2 à 6 unités alkylèneoxy par mole d'alkylglycoside.

3. Utilisation selon l'une quelconque des revendications 1 à 2, dans laquelle le dérivé anionique et/ou le produit d'addition d'oxyde d'alkylène non ionique est utilisé en une quantité de 0,1 à 20 % en poids, sur la base du poids de l'alkylglycoside.

4. Utilisation selon l'une quelconque des revendications 1 à 3 dans laquelle le dérivé anionique est de formule générale 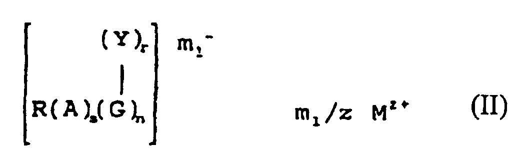 dans laquelle R, A, G, s et n sont tels que ci-dessus, r est un nombre valant 0 ou 1, la somme de tous les r étant d'au moins 1, de préférence 1 à 2, Y est un groupe (OR₁)ₚ(X)ₘ, dans laquelle R₁ est un groupe hydrocarboné aliphatique comportant de 1 à 8, de préférence de 1 à 4 atomes de carbone, m est un nombre allant de 1 à 2, X est un groupe contenant un carboxyle, p vaut 0 ou 1, et m vaut 1, m₁ est la charge totale de l'anion et M est un cation de préférence monovalent, et z est la charge du cation.

5. Utilisation selon l'une quelconque des revendications 1 à 4,
**caractérisée en ce que**
le produit d'addition d'oxyde d'éthylène est de formule 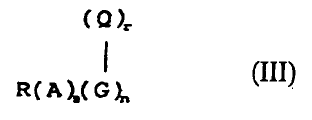 dans laquelle R, A, G, s, n et r sont tels que ci-avant et Q est le groupe (B)ᵥH, où B est un groupe alkylèneoxy et v est un nombre allant de 1 à 12, de préférence de 2 à 6.

6. Utilisation selon l'une quelconque des revendications 1 à 5, dans laquelle R dans le glycoside est le dérivé anionique ou le produit d'addition d'oxyde d'alkylène non ionique sont identiques.

7. Concentré d'un alkylglycoside, **caractérisé en ce qu'**il contient
a) 10 à 80, de préférence 45 à 70 % en poids d'un alkylglycoside de formule I selon la revendication 1,
b) 0,1 à 20, de préférence 0,5 à 15 % en poids, sur la base de la quantité d'alkylglycoside, de l'additif inhibiteur de cristallisation selon la revendication 1, 2, 4, 5 ou 6, et
c) 4 à 90 % en poids d'eau.

8. Concentré selon la revendication 7, **caractérisé en ce que** l'additif inhibiteur de cristallisation consiste en le dérivé anionique, qui a une teneur de 0,1 à 8 % en poids, sur la base de l'alkylglycoside.

9. Concentré selon la revendication 7, **caractérisé en ce que** le dérivé inhibiteur de cristallisation consiste en le produit d'addition d'oxyde d'alkylène non ionique, qui a une teneur de 2 à 20 % en poids, sur la base de l'alkylglycoside.

10. Procédé de prévention ou d'inhibition de la formation d'une cristallisation dans un concentré aqueux d'un alkylglycoside, **caractérisé en ce qu'**on ajoute au concentré, en une quantité de 0,1 à 20 % en poids, sur la base du poids de l'alkylglycoside, un dérivé anionique d'un glycoside
R(A)ₛ(G)ₙ (I)
dans laquelle R est un groupe hydrocarboné aliphatique comportant de 6 à 20, de préférence de 8 à 16 atomes de carbone, G est un résidu saccharide, A est un oxyalkylène comportant de 2 à 4 atomes de carbone ou un 3-oxy-2-hydroxypropylène, n est un nombre allant de 1 à 10, de préférence un nombre allant de 1 à 4, et s est un nombre allant de 0 à 5 ; ou un produit d'addition d'oxyde d'alkylène non ionique, dans lequel l'oxyde d'alkylène contient 2 à 4 atomes de carbone, d'un glycoside de formule I.
